(19) Europäisches Patentamt
European Patent Office
Office européen des brevets

(11) EP 4 671 304 A1

(12) EUROPEAN PATENT APPLICATION

(43) Date of publication:
**31.12.2025 Bulletin 2026/01**

(21) Application number: **25185488.1**

(22) Date of filing: **26.06.2025**

(51) International Patent Classification (IPC):
***C08G 71/02*** (2006.01) ***C07D 317/36*** (2006.01)
***C08G 71/04*** (2006.01) ***C08L 75/02*** (2006.01)
***C08L 75/04*** (2006.01) ***C08L 75/06*** (2006.01)
***C08L 75/08*** (2006.01) ***C09D 175/02*** (2006.01)
***C09D 175/06*** (2006.01) ***C09D 175/08*** (2006.01)

(52) Cooperative Patent Classification (CPC):
**C08G 71/02; C07D 317/36; C08G 71/04; C08L 75/02; C08L 75/04; C08L 75/06; C08L 75/08; C09D 175/02; C09D 175/06; C09D 175/08**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **28.06.2024 US 202418758594**
**28.06.2024 US 202463665254 P**
**31.12.2024 TW 113151656**

(71) Applicant: **Far Eastern New Century Corporation**
**Taipei (TW)**

(72) Inventors:
- **WANG, Cheng-Ting**
**10602 Taipei City (TW)**
- **CHEN, Chia-Huei**
**10602 Taipei City (TW)**

(74) Representative: **Zimmermann, Tankred Klaus**
**Schoppe, Zimmermann, Stöckeler**
**Zinkler, Schenk & Partner mbB**
**Patentanwälte**
**Radlkoferstrasse 2**
**81373 München (DE)**

# (54) CONDENSATION TYPE POLYURETHANE RESIN AND COVERED FIBER INCLUDING THE SAME

(57) A condensation type polyurethane resin includes a first carbamate segment represented by Formula (I), a second carbamate segment represented by Formula (II), and a urea-containing segment represented by Formula (III). The urea-containing segment is present in an amount of greater than 0 mol% and less than 27 mol%, based on a total amount of the first carbamate segment, the second carbamate segment, and the urea-containing segment as 100 mol%. Each of the substituents in Formulae (I) to (III) is given the definitions as set forth in the Specification and Claims. The condensation type polyurethane resin has an amine value of at least 10 meq/kg. A covered fiber, which includes a core and the condensation type polyurethane resin, is also provided.



Processed by Luminess, 75001 PARIS (FR)

## Description

The present disclosure relates to a polyurethane (PU) resin, and more particularly to a condensation type polyurethane resin and a covered fiber including the same.

A conventional polyurethane is generally prepared using isocyanate and polyol as raw materials (e.g., TW I788086 B, US 11414543 B2, and US 20240336720 A1). In the manufacturing process of the conventional polyurethane, not only the isocyanate is itself toxic, but also requires use of another more toxic raw material, which is phosgene. Therefore, the industry actively pursues the concept of green manufacturing in the manufacturing process of non-isocyanate polyurethane (NIPU).

In the concept of green manufacturing, NIPU is mainly manufactured by a poly-addition process or a polycondensation process in which the structures of products obtained are different from each other.

U.S. 20230183423 A1 discloses that NIPU may be prepared by subjecting multiple cyclic carbonate and polyamine to an addition polymerization process. The thus synthesized NIPU has (-OH) functional groups on side chains thereof, and hence is known as polyhydroxyurethane (PHU) with a chemical structure represented by

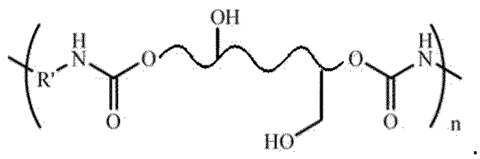

Due to the presence of abundant hydrogen bonding forces between PHU molecules, the PHU is not conducive to being processed into a covered fiber.

In contrast, a condensation polymerization process generally involves reacting diamine and carbonate, followed by a transesterification reaction with polyol so as to obtain NIPU. Thus, the NIPU synthesized by the condensation polymerization process does not have side chains containing (-OH) functional groups, indicating that the chemical structure of such NIPU is different from that of the PHU as described above.

CN 101696271 A, CN 103865059 A, CN 116355210 A, CN 112853531 A, and CN 104513393 B each discloses use of carbonate and diamine to prepare a dicarbamate monomer (i.e., a type of urethane diol), followed by a polymerization reaction with polyol, so as to obtain NIPU suitable for direct melt processing. However, preparation of the dicarbamate monomer requires use of organic solvents for purification by recrystallization, so as to remove impurities (e.g., urea-containing small molecules), causing generation of a large amount of liquid waste that needs to be recycled separately, indicating that such preparation process is energy-consuming and not environmentally friendly. Additionally, a fluidity of the NIPU thus obtained is still not conducive to the processing into the covered fiber.

In addition, as exemplified in methods for preparing NIPU as disclosed in CN 104910348 B and CN 116903854 A, a dimethylcarbamate monomer (i.e., a dicarbamate) reacts with a diol to form a prepolymer terminated with (-OH) functional groups, followed by a polymerization reaction so as to obtain NIPU. Such methods of CN 104910348 B and CN 116903854 A involve multiple complex steps, and a fluidity of the NIPU thus obtained is still not conducive to the processing into the covered fiber.

Furthermore, TW 201634531 A and CN 107400233 B disclose use of carbonate and diamine in a molar ratio of 1:1 to prepare polyurea; however, the chemical structures of polyurea and polyurethane are very different as shown below.
Polyurea:

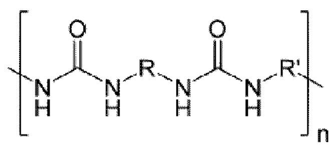

Polyurethane:

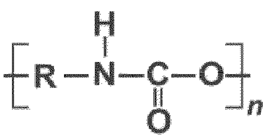

The hydrogen bonding force between polyurea molecules is very strong, resulting in poor fluidity of the polyurea, which is not conducive to the processing into the covered fiber. For smooth processing of the polyurea, use of organic

solvent is usually required. However, use of the organic solvent will incur problems such as industrial safety concerns and recycling of liquid waste.

Although the concept of green manufacturing that is environmentally friendly exists in the preparation of the NIPUs as mentioned above, not all of the NIPUs are suitable for processing into the covered fiber. When NIPU is subsequently used to be processed into the covered fiber, a fluidity of the NIPU still needs to be considered to meet the required processing properties (e.g., a relatively high elongation and a relatively moderate melt flow index (MI)), which may involve addition of other additives (e.g., tackifiers) to enhance coverage ratio of the NIPU.

Therefore, an object of the disclosure is to provide a condensation type polyurethane resin and a covered fiber including the same that can alleviate at least one of the drawbacks of the prior art.

According to an aspect of the disclosure, there is provided the condensation type polyurethane resin according to claim 1.

According to an aspect of the disclosure, there is provided the covered fiber according to claim 10.

Before the present disclosure is described in greater detail, it should be noted that if any prior art publication is referred to herein, such reference does not constitute an admission that the publication forms a part of the common general knowledge in the art, in Taiwan or any other country.

For the purpose of this specification, it will be clearly understood that the word "comprising" means "including but not limited to", and that the word "comprises" has a corresponding meaning.

Unless otherwise defined, all technical and scientific terms used herein have the meaning commonly understood by a person skilled in the art to which the present disclosure belongs. One skilled in the art will recognize many methods and materials similar or equivalent to those described herein, which could be used in the practice of the present disclosure. Indeed, the present disclosure is in no way limited to the methods and materials described.

The present disclosure provides a condensation type polyurethane resin including a first carbamate segment represented by Formula (I), a second carbamate segment represented by Formula (II), and a urea-containing segment represented by Formula (III),

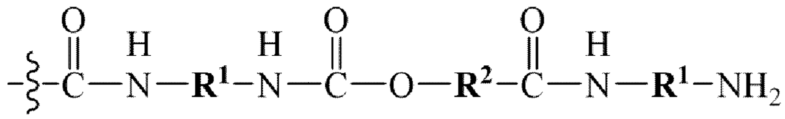

(I)

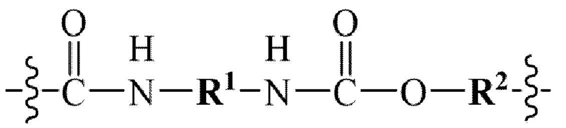

(II)

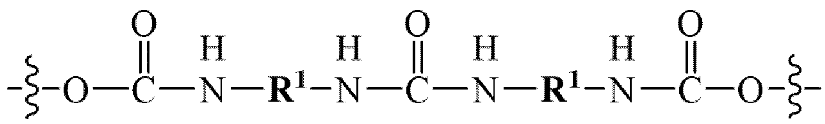

(III).

The condensation type polyurethane resin has an amine value of at least 10 meq/kg.

According to the present disclosure, the condensation type polyurethane resin is a condensation type non-isocyanate polyurethane resin.

The urea-containing segment is present in an amount of greater than 0 mol% and less than 27 mol%, based on a total amount of the first carbamate segment, the second carbamate segment, and the urea-containing segment as 100 mol%.

In each of Formulae (I) to (III), $R^1$ is a hydrocarbon group of $C_2$ to $C_{20}$, and $R^2$ is selected from the group consisting of

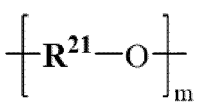

,

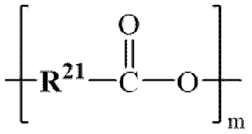, 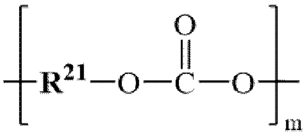, 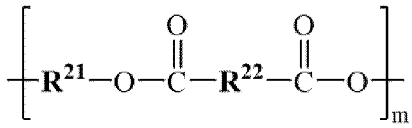,

and combinations thereof, wherein each of $R^{21}$ and $R^{22}$ is independently a hydrocarbon group of $C_2$ to $C_{20}$, and m is an integer ranging from 1 to 250.

In certain embodiment, at least one end of the urea-containing segment is connected to $R^2$.

In certain embodiments, the condensation type non-isocyanate polyurethane resin has an amine value ranging from 16 meq/kg to 30 meq/kg.

In certain embodiments, the condensation type non-isocyanate polyurethane resin has a melt flow index (MI) of not greater than 25.0 g/10 min at 170°C under a load of 2.16 kg. In certain embodiments, the condensation type non-isocyanate polyurethane resin has a melt flow index (MI) of not greater than 10.0 g/10 min at 170°C under a load of 2.16 kg. In certain embodiments, the condensation type non-isocyanate polyurethane resin has a melt flow index (MI) of not greater than 5.0 g/10 min at 170°C under a load of 2.16 kg.

In certain embodiments, the condensation type non-isocyanate polyurethane resin has a melt flow index (MI) of not greater than 65.0 g/10 min at 195°C under a load of 2.16 kg. In certain embodiments, the condensation type non-isocyanate polyurethane resin has a melt flow index (MI) of not greater than 36.0 g/10 min at 195°C under a load of 2.16 kg. In certain embodiments, the condensation type non-isocyanate polyurethane resin has a melt flow index (MI) of not greater than 15.0 g/10 min at 195°C under a load of 2.16 kg.

In certain embodiments, the urea-containing segment is present in an amount ranging from 2 mol% to 25 mol%, based on the total amount of the first carbamate segment, the second carbamate segment, and the urea-containing segment as 100 mol%. In certain embodiments, the urea-containing segment is present in an amount ranging from 12 mol% to 21 mol%, based on the total amount of the first carbamate segment, the second carbamate segment, and the urea-containing segment as 100 mol%. In certain embodiments, the urea-containing segment is present in an amount ranging from 15 mol% to 21 mol%, based on the total amount of the first carbamate segment, the second carbamate segment, and the urea-containing segment as 100 mol%.

In certain embodiments, the condensation type non-isocyanate polyurethane resin has an elongation of not less than 325%.

In certain embodiments, the condensation type non-isocyanate polyurethane resin has an enthalpy of melting ($\Delta H_m$) of not greater than 30 J/g. In certain embodiments, the condensation type non-isocyanate polyurethane resin has an enthalpy of melting ($\Delta H_m$) ranging from 24 J/g to 27 J/g.

In certain embodiments, $R^2$ is selected from the group consisting of

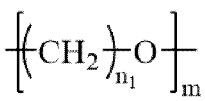, 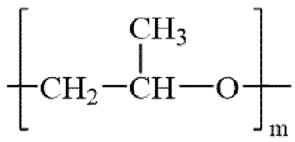, 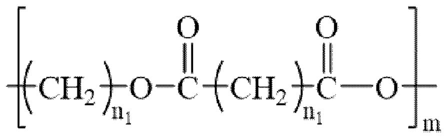,

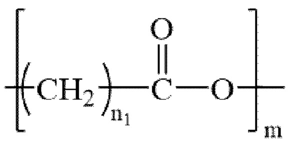, 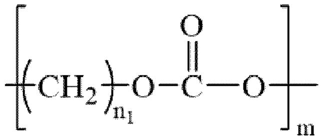, 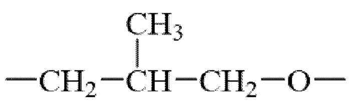,

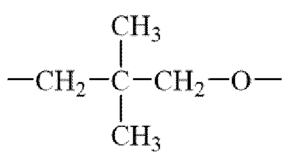, 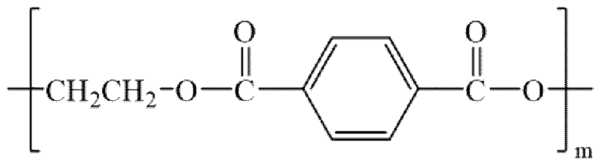,

and combinations thereof, wherein $n_1$ is an integer ranging from 2 to 20, and m is an integer ranging from 1 to 250. In certain embodiments, $R^2$ includes at least two segments selected from the group consisting of

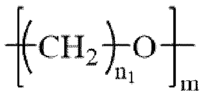

,

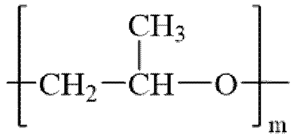

,

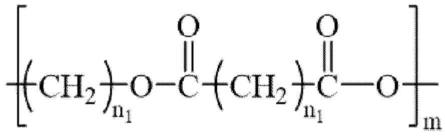

,

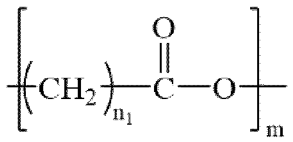

,

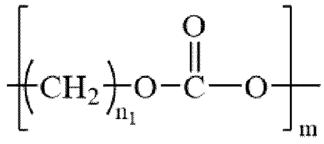

,

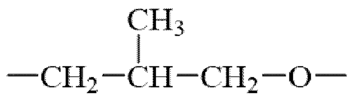

,

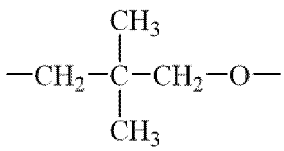

, and

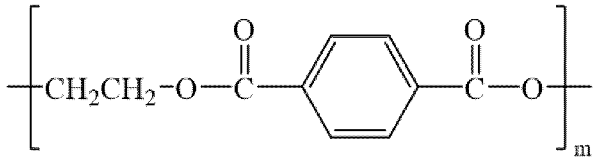

.

The present disclosure also provides a method for preparing the condensation type non-isocyanate polyurethane resin which includes subjecting a diamine compound and a carbonate compound to a carbamation reaction so as to form a carbamate mixture that includes an amine hydrocarbamate and a hydrocarbon dicarbamate diester, and then subjecting the carbamate mixture and a diol compound to a polycondensation reaction so as to form the condensation type non-isocyanate polyurethane resin.

In certain embodiments, the diamine compound is selected from the group consisting of cyclic diamines, acyclic diamines, and a combination thereof. In certain embodiments, the diamine compound may be selected from the group consisting of pentamethylene diamine (PDA), hexamethylene diamine, decanediamine, m-phenylenediamine, m-xylenediamine (MXDA), 1,3-bis(aminomethyl)cyclohexane, and combinations thereof.

In certain embodiments, the diol compound is selected from the group consisting of cyclic diol, acyclic diol, and a combination thereof. In certain embodiments, the diol compound is selected from the group consisting of ether diol (e.g., polyalkyl diol), ester diol (e.g., polyester diol), carbonate diol (e.g., polycarbonate diol), and alkyl diol (e.g., alkylene diol). In certain embodiments, the diol compound is selected from the group consisting of polyethylene glycol (PEG), poly(tetramethylene ether) glycol (PTMEG), polypropylene glycol (PPG), poly(propylene-co-ethylene) glycol (PPG-co-PEG), polybutylene adipate (PBA), poly(1,6-hexamethylene adipate) (PHA), poly(ethylene adipate) (PEA), poly(butylene succinate) (PBS), poly(ε-caprolactone) (PCL), bis(2-hydroxyethyl)terephthalate (BHET) oligomer, poly(hexamethylene carbonate) diol (PHC diol), polyethylene carbonate (PEC), polypropylene carbonate (PPC), ethylene glycol (EG), 1,2-propylene glycol (PG), 1,4-butanediol (BDO), neopentyl glycol (NPG), 2-methyl-1,3-propanediol (MPDO), diethylene glycol (DEG), triethylene glycol (TEG), bis(2-hydroxyethyl)terephthalate (BHET), 1,4-cyclohexanedimethanol, isosorbide (ISOS), 2,2,4,4-tetramethyl-1,3-cyclobutanediol (TMCD), and combinations thereof.

In certain embodiments, the diol compound is a combination of at least two of the diols in the group mentioned above. In certain embodiments, the diol compound is a combination of two of the diols in the group mentioned above, and a molar ratio of the two of the diols ranges from 1.0:0.3 to 1.0:3.0.

In certain embodiments, the diol compound is a combination of the poly(tetramethylene ether) glycol (PTMEG) and the poly(hexamethylene carbonate) diol (PHC diol). A molar ratio of the PTMEG to the PHC diol ranges from 1.0:0.3 to 1.0:3.0.

In certain embodiments, the diol compound has a number average molecular weight ($M_n$) ranging from 60 to 10000. In certain embodiments, the diol compound has a number average molecular weight ($M_n$) ranging from 60 to 6000.

The present disclosure also provides a covered fiber which includes a core and the aforesaid condensation type polyurethane resin that covers the core. The condensation type polyurethane resin is the condensation type non-isocyanate polyurethane resin as described above.

Examples of the material of the core may include, but are not limited to, natural fiber, polyolefin fiber, acetate fiber, polyester fiber, polyamide fiber, polyurethane fiber, glass fiber, carbon fiber, or combinations thereof. In certain embodiments, the polyamide fiber may be selected from the group consisting of aramid fiber, aliphatic polyamide fiber,

polyphthalamide fiber, and combinations thereof.

Examples of equipment that can be used to prepare the covered fiber of the present disclosure may include, but are not limited to, a co-extrusion machine and a coating machine. Specifically, the procedures for using the co-extrusion machine to prepare the covered fiber of the present disclosure may include introducing the core in a fibrous form into the co-extrusion machine, followed by injecting the condensation type non-isocyanate polyurethane resin in a molten state into the co-extrusion machine, so that the condensation type non-isocyanate polyurethane resin covers the core during a co-extrusion process.

According to the present disclosure, the condensation type non-isocyanate polyurethane resin may be applied in various fields, e.g., automobiles, toys, biomedical products, electronic products, shoe materials (i.e., the entire shoe including outsole, midsole, insole, upper, etc.), sports equipment, textiles (e.g., fiber or film), and other daily necessities, or in food and construction industries, and may be used in coatings, adhesives, wires and cables, etc. It should be noted that in order to be applied in different fields, the condensation type non-isocyanate polyurethane resin may be required to have different properties. In this regard, the condensation type non-isocyanate polyurethane resin of the present disclosure suitable for applications in different fields may be prepared by selecting different types of the diamine compound, the carbonate compound, or the diol compound.

According to the present disclosure, the condensation type non-isocyanate polyurethane resin may have a relatively high elongation and a relatively moderate melt flow index (MI), and can exhibit excellent coverage ratio in a process of manufacturing the covered fiber without the need for additional tackifiers or solvents.

The disclosure will be further described by way of the following examples. However, it should be understood that the following examples are solely intended for the purpose of illustration and should not be construed as limiting the disclosure in practice.

**Preparation of condensation type non-isocyanate polyurethane resin**

**Example 1 (E1)**

First, hexane-1,6-diamine (HMDA) (purchased from Huafon Chemical Co., Ltd., serving as a diamine compound), ethylene carbonate (EC) (purchased from Oriental Union Chemical Corporation, serving as a carbonate compound), poly(tetramethylene ether) glycol 1000 (PTMEG 1000) (purchased from Formosa Asahi Spandex Co., Ltd., serving as a diol compound), and poly(hexamethylene carbonate) diol 1000 (PHC diol 1000) (purchased from Tosoh Corporation, model no: Nippollan 981, serving as a diol compound) were mixed at a molar ratio of 1.0:2.1:0.2:0.1 in a 1 L stainless steel reactor, and then subjected to a carbamation reaction at 75°C under stirring for a time period of 6.0 hours, so as to obtain a carbamate mixture of E1 that had an amine value of 2.1 mg KOH/g, which was determined according to the procedures set forth in ASTM D2074.

After adding stannous chloride ($SnCl_2$) (serving as a catalyst) into the aforesaid reactor, a polycondensation reaction was conducted at 180°C and at a pressure of not greater than 30 torr for a time period of 1 hour, followed by controlling the pressure by reduction of the same to 3 torr until the product viscosity no longer increased, so as to obtain a condensation type non-isocyanate polyurethane resin of E1 that had an enthalpy of melting of 20 J/g.

**Examples 2 to 8 (E2 to E8)**

The procedures and conditions for preparing the condensation type non-isocyanate polyurethane resins of E2 to E8 were substantially similar to those of E1, except that: the time periods for conducting the carbamation reaction in E2 to E8 were respectively changed to 4.1 hours (for E2), 3.9 hours (for E3), 3.7 hours (for E4), 3.6 hours (for E5), 3.2 hours (for E6), 3.1 hours (for E7), and 3.0 hours (for E8); the HMDA, EC, PTMEG 1000, and PHC diol 1000 in E4, E6 and E8 were respectively mixed at molar ratios of 1.0:2.1:0.05:0.15 (for E4), 1.0:2.1:0.15:0.05 (for E6), and 1.0:2.1:0.05:0.1 (for E8); the carbamate mixtures of E2 to E8 respectively had amine values of 12.9 mg KOH/g (for E2), 14.5 mg KOH/g (for E3), 17.2 mg KOH/g (for E4), 18.5 mg KOH/g (for E5), 19.9 mg KOH/g (for E6), 21.3 mg KOH/g (for E7), and 23.8 mg KOH/g (for E8), which were determined according to the procedures set forth in ASTM D2074; and the condensation type non-isocyanate polyurethane resins of E2 to E8 thus obtained respectively had enthalpies of melting of 27 J/g (for E2), 27 J/g (for E3), 26 J/g (for E4), 25 J/g (for E5), 26 J/g (for E6), 24 J/g (for E7), and 27 J/g (for E8).

**Example 9 (E9)**

The procedures and conditions for preparing the condensation type non-isocyanate polyurethane resin of E9 were substantially similar to those of E1, except that: in E9, the PHC diol 1000 was replaced with the PTMEG 1000; the HMDA, EC, and PTMEG 1000 were mixed at a molar ratio of 1.0:2.1:0.2; the carbamate mixture of E9 had an amine value of 1.9 mg KOH/g, which was determined according to the procedures set forth in ASTM D2074; and the condensation type

non-isocyanate polyurethane resin of E9 thus obtained had an enthalpy of melting of 9 J/g.

**Example 10 (E10)**

The procedures and conditions for preparing the condensation type non-isocyanate polyurethane resin of E10 were substantially similar to those of E1, except that: in E10, the PHC diol 1000 was replaced with the PTMEG 1000; the HMDA, EC, and PTMEG 1000 were mixed at a molar ratio of 1.0:2.1:0.3; the carbamate mixture of E10 had an amine value of 2.3 mg KOH/g, which was determined according to the procedures set forth in ASTM D2074; and the condensation type non-isocyanate polyurethane resin of E10 thus obtained had an enthalpy of melting of 15 J/g.

**Example 11 (E11)**

The procedures and conditions for preparing the condensation type non-isocyanate polyurethane resin of E11 were substantially similar to those of E1, except that: in E11, the PHC diol 1000 was replaced with bis(2-hydroxyethyl) terephthalate (BHET) (purchased from Tokyo Chemical Industry Co., Ltd.); the HMDA, EC, PTMEG 1000, and BHET were mixed at a molar ratio of 1.0:2.1:0.1:0.1; the carbamate mixture of E11 had an amine value of 1.8 mg KOH/g, which was determined according to the procedures set forth in ASTM D2074; and the condensation type non-isocyanate polyurethane resin of E11 thus obtained had an enthalpy of melting of 12 J/g.

**Example 12 (E12)**

The procedures and conditions for preparing the condensation type non-isocyanate polyurethane resin of E12 were substantially similar to those of E9, except that: in E12, the PTMEG 1000 was replaced with poly(ε-caprolactone) 2000 (PCL 2000) (purchased from Huafon Chemical Co., Ltd.); the HMDA, EC, and PCL 2000 were mixed at a molar ratio of 1.0:2.1:0.2; the carbamate mixture of E12 had an amine value of 1.8 mg KOH/g, which was determined according to the procedures set forth in ASTM D2074; and the condensation type non-isocyanate polyurethane resin of E12 thus obtained had an enthalpy of melting of 23 J/g.

**Example 13 (E13)**

The procedures and conditions for preparing the condensation type non-isocyanate polyurethane resin of E13 were substantially similar to those of E1, except that: in E13, the PTMEG 1000 was replaced with the PHC diol 1000; the HMDA, EC, and PHC diol 1000 were mixed at a molar ratio of 1.0:2.1:0.2, and then subjected to the carbamation reaction at 75°C under stirring for the time period of 2.8 hours to obtain the carbamate mixture of E13 having an amine value of 29.5 mg KOH/g, which was determined according to the procedures set forth in ASTM D2074, followed by the polycondensation reaction, so as to obtain the condensation type non-isocyanate polyurethane resin of E13 that had an enthalpy of melting of 21 J/g.

**Example 14 (E14)**

The procedures and conditions for preparing the condensation type non-isocyanate polyurethane resin of E14 were substantially similar to those of E1, except that: in E14, the HMDA was replaced with pentamethylene diamine (PDA) (purchased from Cathay Biotech Inc.); the PDA, EC, PTMEG 1000, and PHC diol 1000 were mixed at a molar ratio of 1.0:2.1:0.1:0.1, and then subjected to the carbamation reaction at 75°C under stirring for the time period of 3.5 hours to obtain the carbamate mixture of E14 having an amine value of 17.4 mg KOH/g, which was determined according to the procedures set forth in ASTM D2074, followed by the polycondensation reaction, so as to obtain the condensation type non-isocyanate polyurethane resin of E14 that had an enthalpy of melting of 24 J/g.

**Example 15 (E15)**

The procedures and conditions for preparing the condensation type non-isocyanate polyurethane resin of E15 were substantially similar to those of E1, except that: in E15, the HMDA was replaced with m-xylenediamine (MXDA) (purchased from Mitsubishi Chemical Infratec Co., Ltd.); the MXDA, EC, PTMEG 1000, and PHC diol 1000 were mixed at a molar ratio of 1.0:2.1:0.1:0.2, and then subjected to the carbamation reaction at 75°C under stirring for the time period of 3.9 hours to obtain the carbamate mixture of E15 having an amine value of 16.9 mg KOH/g, which was determined according to the procedures set forth in ASTM D2074, followed by the polycondensation reaction, so as to obtain the condensation type non-isocyanate polyurethane resin of E15 that had an enthalpy of melting of 25 J/g.

**Comparative Examples 1 and 2 (CE1 and CE2)**

The procedures and conditions for preparing the condensation type non-isocyanate polyurethane resins of CE1 and CE2 were substantially similar to those of E1, except that: the time periods for conducting the carbamation reaction in CE1 and CE2 were respectively changed to 2.9 hours (for CE1) and 2.8 hours (for CE2); the carbamate mixtures of CE1 and CE2 respectively had amine values of 25.4 mg KOH/g (for CE1) and 29.0 mg KOH/g (for CE2), which were determined according to the procedures set forth in ASTM D2074; and the condensation type non-isocyanate polyurethane resins of CE1 and CE2 thus obtained respectively had enthalpies of melting of 35 J/g (for CE1) and 36 J/g (for CE2).

**Comparative Example 3 (CE3)**

The procedures and conditions for preparing the condensation type non-isocyanate polyurethane resin of CE3 were substantially similar to those of E9, except that: in CE3, the HMDA was replaced with the MXDA; the MXDA, EC, and PTMEG 1000 were mixed at a molar ratio of 1.0:2.1:0.2, and then subjected to the carbamation reaction at 75°C under stirring for the time period of 5.0 hours to obtain the carbamate mixture of CE3 having an amine value of 3.2 mg KOH/g, which was determined according to the procedures set forth in ASTM D2074, followed by the polycondensation reaction, so as to obtain the condensation type non-isocyanate polyurethane resin of CE3 that had an enthalpy of melting of 3 J/g.

**Comparative Example 4 (CE4)**

First, the HMDA and EC were mixed at a molar ratio of 1.0:2.1 in a 1 L stainless steel reactor, and then subjected to a carbamation reaction at 75°C under stirring for 6.0 hours, so as to obtain a carbamylated product. Next, the carbamylated product was subjected to washing, filtration (to remove liquids therein) and recrystallization steps in sequence, so as to obtain an intermediate product (in a solid form) mainly composed of di(2-hydroxyethyl) hexamethylene dicarbamate having an amine value of 0.2 mg KOH/g, which was determined according to the procedures set forth in ASTM D2074. Afterwards, the intermediate product, PTMEG 1000, and PHC diol 1000 were mixed at a molar ratio of 1.0:0.1:0.1 to obtain a carbamate mixture of CE4 having an amine value of 0.1 mg KOH/g, which was determined according to the procedures set forth in ASTM D2074, followed by a polycondensation reaction conducted using the procedures and conditions as described in E1, so as to obtain a condensation type non-isocyanate polyurethane resin of CE4 that had an enthalpy of melting of 9 J/g.

**Comparative Examples 5 and 6 (CE5 and CE6)**

The procedures and conditions for preparing the condensation type non-isocyanate polyurethane resins of CE5 and CE6 were substantially similar to those of CE4, except that: in CE5 and CE6, the intermediate product prepared as described in CE4 and the PTMEG 1000 were mixed at molar ratios of 1.0:0.2 (for CE5) and 1.0:0.3 (for CE6) to obtain the carbamate mixtures of CE5 and CE6 each having an amine value of 0.05 mg KOH/g, which was determined according to the procedures set forth in ASTM D2074, followed by the polycondensation reaction conducted using the procedures and conditions as described in E1, so as to obtain the condensation type non-isocyanate polyurethane resins of CE5 and CE6 that respectively had enthalpies of melting of 12 J/g (for CE5) and 9 J/g (for CE6).

**Comparative Example 7 (CE7)**

The procedures and conditions for preparing the condensation type non-isocyanate polyurethane resin of CE7 were substantially similar to those of E10, except for the following differences: the EC was replaced with dimethyl carbonate (DMC) (purchased from Emperor Chemical Co., Ltd.); the HMDA and DMC were first reacted to obtain an intermediate product mainly composed of hexamethylene dicarbamate, which were then subjected to a methanol elimination reaction with 1,4-butanediol (BDO) (purchased from Emperor Chemical Co., Ltd.) under a nitrogen environment at 160°C for a time period of 2 hours, followed by addition of PTMEG 1000, such that a molar ratio of the HMDA, DMC, BDO and PTMEG 1000 was 1:10:4:0.26; the carbamate mixture of CE7 obtained after the carbamation reaction was subjected to the polycondensation reaction, so as to obtain the condensation type non-isocyanate polyurethane resin of CE7 that had an enthalpy of melting of 10 J/g.

**Preparation of polyurethane resin**

**Comparative Example 8 (CE8)**

Thermoplastic polyurethane (TPU) pellets purchased from Coating P. Materials Co., Ltd. (model no: no.:

BE-5038E), which were obtained by reacting poly(tetramethylene ether) glycol (PTMEG) and methylene diphenyl diisocyanate (MDI), directly serve as a polyurethane resin of CE8. The polyurethane resin of CE8 is a type of polyurethane prepared using an isocyanate.

**Property evaluation**

Each of the condensation type non-isocyanate polyurethane resins of E1 to E15 and CE1 to CE7 and the polyurethane resin of CE8 was subjected to measurements described below. The results for the measurements are listed in Table 1 below.

1. Measurement of amine value

Each of the condensation type non-isocyanate polyurethane resins of E1 to E15 and CE1 to CE7 and the polyurethane resin of CE8 was subjected to measurement of amine value according to the procedures set forth in ASTM D2074.

2. Measurement of content of urea-containing segment

Each of the condensation type non-isocyanate polyurethane resins of E1 to E15 and CE1 to CE7 and the polyurethane resin of CE8 was subjected to hydrogen-1 nuclear magnetic resonance ($^1H$ NMR) spectroscopy (in DMSO-$d_6$) to obtain a $^1H$ NMR spectrum, followed by calculating a first integrated area ($A_1$) of the resonance signal with chemical shifts ranging from 5.6 ppm to 5.8 ppm and a second integrated area ($A_2$) of the resonance signal with chemical shifts ranging from 6.7 ppm to 7.3 ppm.

The content of urea-containing segment in each of the condensation type non-isocyanate polyurethane resins of E1 to E15 and CE1 to CE7 and the polyurethane resin of CE8 was then calculated using the following Equation (1):

$$C = [A_1/(A_1+A_2)] \times 100\% \qquad (1)$$

where

C= content of urea-containing segment (mol%)
$A_1$= first integrated area
$A_2$= second integrated area

3. Measurement of melt flow index (MI)

Each of the condensation type non-isocyanate polyurethane resins of E1 to E15 and CE1 to CE7 and the polyurethane resin of CE8 was divided into 5 parts and then subjected to measurement of melt flow index (MI) at 195°C and 170°C under a load of 2.16 kg using a melt flow indexer (manufacturer: Gotech Testing Machines Inc., model no.: GT-7100-MIB) according to the procedures set forth in ASTM D1238 (published in 2010). The results (i.e., average MI) are listed in Table 1 below.

4. Measurement of elongation

Elongation was measured according to the procedures set forth in ASTM D412. Briefly, each of the condensation type non-isocyanate polyurethane resins of E1 to E15 and CE1 to CE7 was pressed into a sheet with a thickness of 1 mm, and then cut into five dumbbell-shaped specimens of type C. Thereafter, the five dumbbell-shaped specimens were subjected to measurement of elongation using a universal testing machine (manufacturer: Instron Ltd., model no.: 5566) at a tensile speed of 500 ± 50 mm/min with a gauge length of 64.2 mm. The results (i.e., average elongation) are listed in Table 1 below.

5. Measurement of melting temperature ($T_m$) and enthalpy of melting ($\Delta H_m$)

Each of the condensation type non-isocyanate polyurethane resins of E1 to E15 and CE1 to CE7 and the polyurethane resin of CE8 was subjected to a first heating treatment (from -70°C to 200°C), a first cooling treatment (from 200°C to -70°C), and a second heating treatment (from -70°C to 200°C) in sequence using a differential scanning calorimeter (DSC, manufacturer: Hitachi High-Tech Corporation) at a rate of 10°C/min. The melting temperature ($T_m$) was

recorded during the second heating treatment (as listed in Table 1 below), and the enthalpy of melting ($\Delta H_m$) was calculated (as described in the respective Examples and Comparative Examples above).

6. Preparation of covered fiber

Each of the condensation type non-isocyanate polyurethane resins of E1 to E15 and CE1 to CE7 and the polyurethane resin of CE8 was dried to have a moisture content of less than 1000 ppm, followed by feeding into an extruder for melting the thus dried condensation type non-isocyanate polyurethane resin or polyurethane resin at a temperature ranging from 150°C to 200°C under a pressure ranging from 30 kgf to 120 kgf. Then, the thus molten condensation type non-isocyanate polyurethane resin or polyurethane resin was extruded to cover surfaces of multiple polyethylene terephthalate (PET) fibers (serving as cores), followed by cooling and winding in sequence, so as to obtain a covered fiber of a respective one of E1 to E15 and CE1 to CE8.

7. Evaluation of coverage ratio

The covered fibers of E1 to E15 and CE1 to CE8 were cut, followed by sputtering with gold on the cross-sections thereof to serve as samples (n=50). Afterwards, each of the samples were examined using a scanning electron microscope (SEM, manufacturer: JEOL Ltd., model no.: JSM-6390LV) to observe whether the cores in the cross-sections were completely covered with the condensation type non-isocyanate polyurethane resin or polyurethane resin, and coverage ratio on the cores was then evaluated according to the following criteria:

◎: the proportion of the sample in which the cores were completely covered was not less than 95%;

o : the proportion of the sample in which the cores were completely covered was not less than 75% and less than 95%;

△ : the proportion of the sample in which the cores were completely covered was not less than 50% and less than 75%; and

× : the proportion of the sample in which the cores were completely covered was less than 50%.

Table 1

| | Amine value of polyurethane resin (meq/kg) | Content of urea-containing segment (mol%) | MI at 195°C (g/10 min) | MI at 170°C (g/10 min) | Elongation (%) | $T_m$ (°C) | Coverage ratio |
|---|---|---|---|---|---|---|---|
| E1 | 10 | 2 | 56.3 | 14.3 | 580 | 94 | ○ |
| E2 | 15 | 11 | 50.2 | 12.3 | 545 | 96 | ○ |
| E3 | 19 | 15 | 25.4 | 8.1 | 342 | 121 | ◎ |
| E4 | 19 | 16 | 25.5 | 8.2 | 392 | 116 | ◎ |
| E5 | 20 | 17 | 32.5 | 8.8 | 368 | 119 | ◎ |
| E6 | 25 | 20 | 24.1 | 8.2 | 370 | 114 | ◎ |
| E7 | 30 | 21 | 20.7 | 7.5 | 379 | 113 | ◎ |
| E8 | 34 | 25 | 60.3 | 14.9 | 352 | 120 | ○ |
| E9 | 22 | 17 | 52.2 | 13.1 | 773 | 145 | ○ |
| E10 | 33 | 24 | 51.1 | 13.9 | 950 | 105 | ○ |
| E11 | 18 | 16 | 35.3 | 9.5 | 616 | 149 | ◎ |
| E12 | 25 | 20 | 40.4 | 11.1 | 604 | 45 | ○ |
| E13 | 17 | 13 | 39.1 | 10.8 | 325 | 99 | ○ |
| E14 | 17 | 16 | 26.2 | 8.3 | 376 | 80 | ◎ |
| E15 | 16 | 16 | 22.3 | 7.2 | 539 | 68 | ◎ |

(continued)

| | Amine value of polyurethane resin (meq/kg) | Content of urea-containing segment (mol%) | MI at 195°C (g/10 min) | MI at 170°C (g/10 min) | Elongation (%) | $T_m$ (°C) | Coverage ratio |
|---|---|---|---|---|---|---|---|
| CE1 | 35 | 27 | 80.5 | 29.9 | 293 | 127 | × |
| CE2 | 40 | 29 | 222.3 | 70.5 | 149 | 123 | × |
| CE3 | 45 | 38 | 100.2 | 40.3 | 123 | 51 | × |
| CE4 | 2.2 | 0 | 78.3 | 28.5 | 290 | 95 | × |
| CE5 | 4.7 | 15 | 83.3 | 30.5 | 201 | 146 | × |
| CE6 | 6.5 | 18 | 79.6 | 29.2 | 253 | 105 | × |
| CE7 | 8.1 | 23 | 71.5 | 28.8 | 297 | 139 | × |
| CE8 | 6.5 | 8 | 2.1 | 0 | - | 161 | × |
| "-": Not determined | | | | | | | |

Referring to Table 1, the condensation type non-isocyanate polyurethane resin of a respective one of E1 to E15 exhibited the amine value of not less than 10 meq/kg, the content of the urea-containing segment ranging from 2 mol% to 25 mol%, the MI at 195°C ranging from 20.7 g/10 min to 60.3 g/10 min, the MI at 170°C of not greater than 14.9 g/10 min, the elongation of not less than 325%, and the coverage ratio thereof on the cores rated as either ◎ or ○. These results demonstrate that by virtue of controlling the content of the urea-containing segment of the condensation type non-isocyanate polyurethane resin within a range of 2 mol% to 25 mol%, the condensation type non-isocyanate polyurethane resin is capable of exhibiting a relatively moderate MI, which facilitates complete core coverage in the process of manufacturing the covered fiber.

Referring back to Table 1, the condensation type non-isocyanate polyurethane resin of a respective one of CE1 to CE3 exhibited the content of the urea-containing segment of not less than 27 mol%, the condensation type non-isocyanate polyurethane resin of CE4 exhibited the content of the urea-containing segment of 0 mol%, the condensation type non-isocyanate polyurethane resin of a respective one of CE5 to CE7 exhibited the amine value of not greater than 8.1 meq/kg, the condensation type non-isocyanate polyurethane resin of a respective one of CE1 to CE7 exhibited the melt flow indexes at 195°C and at 170°C of not less than 71.5 g/10 min and 28.5 g/10 min, respectively, the condensation type non-isocyanate polyurethane resin of the respective one of CE1 to CE7 exhibited the elongation of not greater than 297%, and the condensation type non-isocyanate polyurethane resin of the respective one of CE1 to CE7 exhibited the coverage ratio thereof on the cores rated as ×. These results indicate that when the content of the urea-containing segment of the condensation type non-isocyanate polyurethane resin is 0 mol% or not less than 27 mol%, or when the amine value of the condensation type non-isocyanate polyurethane resin is not greater than 8.1 meq/kg, the condensation type non-isocyanate polyurethane resin is capable of exhibiting a relatively high MI (i.e., flows too rapidly) and a relatively low elongation, which tends to cause resin dripping in the process of manufacturing the covered fiber, thereby hindering complete core coverage.

Referring to Table 1 again, the polyurethane resin of CE8 exhibited the amine value of 6.5 meq/kg, the content of the urea-containing segment of 8 mol%, the MI at 195°C of 2.1 g/10 min, the MI at 170°C of 0 g/10 min, the $T_m$ of 161°C, and the coverage ratio thereof on the cores rated as ×. These results demonstrate that the polyurethane resin of CE8 exhibits an extremely low MI (i.e., poor flowability), which makes the process of manufacturing the covered fiber (i.e., melt, extrusion, and covering) difficult to perform at the temperature ranging from 150°C to 200°C, and hence requires a relatively high temperature for processing or the use of additional solvents to improve the process.

Summarizing the above test results, it is clear that the condensation type non-isocyanate polyurethane resin of the present disclosure has a relatively high elongation and a relatively moderate MI, such that the condensation type non-isocyanate polyurethane resin is suitable to be processed into the covered fiber at the temperature ranging from 150°C to 200°C, as well as exhibiting excellent coverage ratio in the process of manufacturing the covered fiber without the need for additional tackifiers or solvents.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of

the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, the one or more features may be singled out and practiced alone without the another one or more features or specific details. It should be further noted that one or more features or specific details from one embodiment may be practiced together with one or more features or specific details from another embodiment, where appropriate, in the practice of the disclosure.

## Claims

1. A condensation type polyurethane resin, **characterized by**:

a first carbamate segment represented by Formula (I), a second carbamate segment represented by Formula (II), and a urea-containing segment represented by Formula (III);

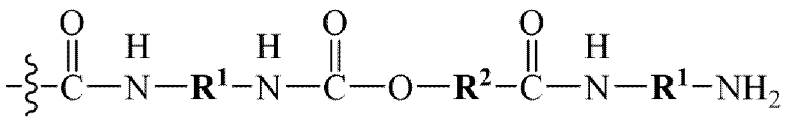

(I)

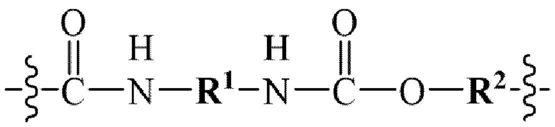

(II)

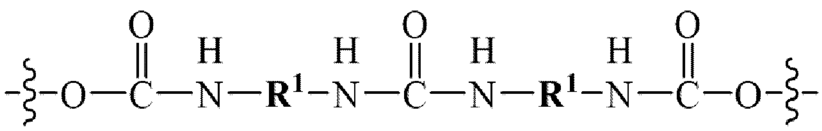

(III)

wherein the urea-containing segment is present in an amount of greater than 0 mol% and less than 27 mol%, based on a total amount of the first carbamate segment, the second carbamate segment, and the urea-containing segment as 100 mol%;
wherein in each of Formulae (I) to (III),
$R^1$ is a hydrocarbon group of $C_2$ to $C_{20}$, and
$R^2$ is selected from the group consisting of

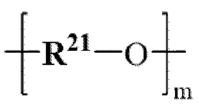

,

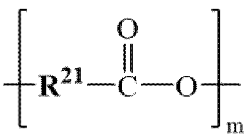

,

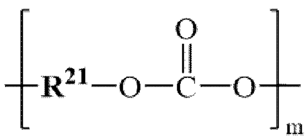

,

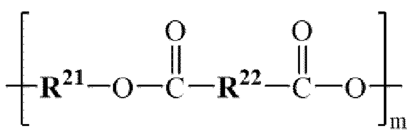

,

and combinations thereof,
wherein

each of $R^{21}$ and $R^{22}$ is independently a hydrocarbon group of $C_2$ to $C_{20}$, and
m is an integer ranging from 1 to 250; and

the condensation type polyurethane resin having an amine value of at least 10 meq/kg.

2. The condensation type polyurethane resin as claimed in claim 1, which has a melt flow index of not greater than 25.0 g/10 min at 170°C under a load of 2.16 kg.

3. The condensation type polyurethane resin as claimed in claim 1, which has a melt flow index of not greater than 65.0 g/10 min at 195°C under a load of 2.16 kg.

4. The condensation type polyurethane resin as claimed in any one of claims 1 to 3, wherein the urea-containing segment is present in an amount ranging from 2 mol% to 25 mol%, based on the total amount of the first carbamate segment, the second carbamate segment, and the urea-containing segment as 100 mol%.

5. The condensation type polyurethane resin as claimed in claim 4, wherein the urea-containing segment is present in an amount ranging from 15 mol% to 21 mol%, based on the total amount of the first carbamate segment, the second carbamate segment, and the urea-containing segment as 100 mol%.

6. The condensation type polyurethane resin as claimed in any one of claims 1 to 5, which has an elongation of not less than 325%.

7. The condensation type polyurethane resin as claimed in any one of claims 1 to 6, which has an enthalpy of melting of not greater than 30 J/g.

8. The condensation type polyurethane resin as claimed in any one of claims 1 to 7, wherein $R^2$ is selected from the group consisting of

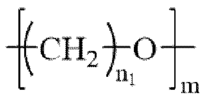
,

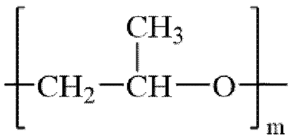
,
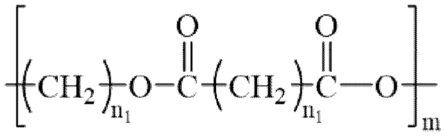
,

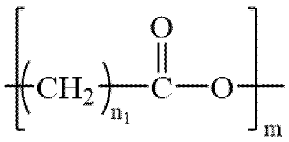
,
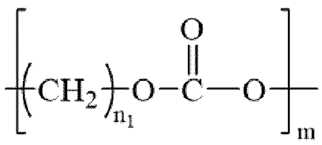
,
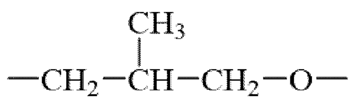
,

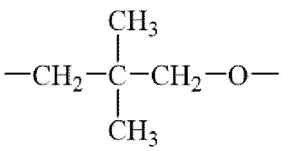
,
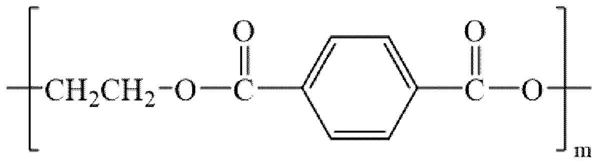
,

and combinations thereof,
wherein

$n_1$ is an integer ranging from 2 to 20, and
m is an integer ranging from 1 to 250.

9. The condensation type polyurethane resin as claimed in claim 8, wherein $R^2$ includes at least two segments selected from the group consisting of

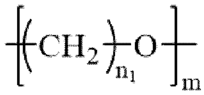, 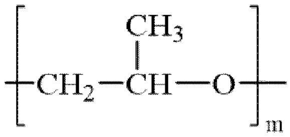, 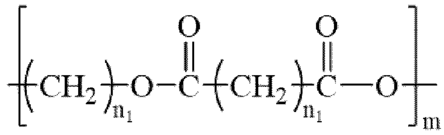,

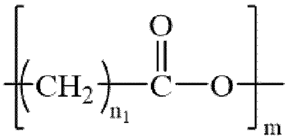, 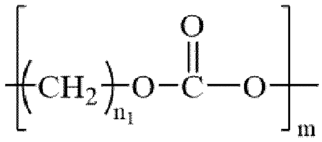, 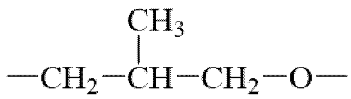,

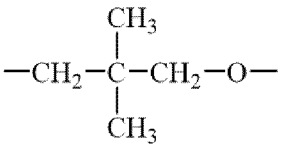, and 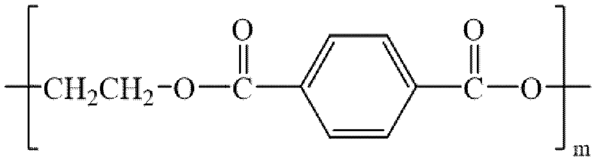.

**10.** A covered fiber, **characterized by**:

a core; and
the condensation type polyurethane resin as claimed in any one of claims 1 to 9 that covers the core.

Europäisches Patentamt
European Patent Office
Office européen des brevets

# EUROPEAN SEARCH REPORT

Application Number
EP 25 18 5488

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHEN ZIYUN ET AL: "A comparison of non-isocyanate and HDI-based poly(ether urethane): Structure and properties", POLYMER, vol. 175, 1 June 2019 (2019-06-01), pages 186-194, XP093326623, AMSTERDAM, NL ISSN: 0032-3861, DOI: 10.1016/j.polymer.2019.05.010 * pages 187-191, points 2.1 Materials and 2.2 Synthesis of NIPU, tables 1-3, ex. NIPU30 and NIPU40, schemes 1 and 2, Fig. 1 and 2, ex. NIPU30 and NIPU40; page 193, conclusion part * ----- | 1-10 | INV. C08G71/02 C07D317/36 C08G71/04 C08L75/02 C08L75/04 C08L75/06 C08L75/08 C09D175/02 C09D175/06 C09D175/08 |
| X | CN 112 011 049 A (INST CHEMISTRY CAS) 1 December 2020 (2020-12-01) * pages 10-13, par, 0045-46, ex. 1, 2, 4, 5, claim 9 * ----- | 1-9 | |
| | | | TECHNICAL FIELDS SEARCHED (IPC) |
| | | | C08G C07D C08L C09D |

The present search report has been drawn up for all claims

| Place of search | Date of completion of the search | Examiner |
|---|---|---|
| Munich | 20 October 2025 | Stefaniu, Cristina |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 25 18 5488

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2025

| Patent document cited in search report | Publication date | Patent family member(s) | Publication date |
|---|---|---|---|
| CN 112011049 A | 01-12-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 11414543 B2
- US 20240336720 A1
- US 20230183423 A1
- CN 101696271 A
- CN 103865059 A
- CN 116355210 A
- CN 112853531 A
- CN 104513393 B
- CN 104910348 B
- CN 116903854 A
- TW 201634531 A
- CN 107400233 B